# EUROPEAN PATENT APPLICATION

(11) **EP 1 764 359 A1**
(43) Date of publication of application: **21.03.2007**
(21) Application number: 05020206.8
(22) Date of filing: 16.09.2005
(51) Int. Cl.: C07C 403/14, C07D 301/26, C07D 303/48

(54) **Process for the preparation of glycidic ester and an aldehyde**

(71) Applicant: DSM IP Assets B.V., 6411 TE Heerlen (NL)
(72) Inventor: Stangl, Jochen, 79664 Wehr (DE); Stritt, Claude, 4056 Basel (CH)
(74) Representative: Pressner, Dietmar

(57) **Abstract**

The invention relates to a process comprising a condensation step wherein a starting aldehyde or ketone is made to react with an ester of an α-haloacid to form a glycidic ester, whereby the reaction is carried out in the presence of a dipolar aprotic compound selected from the group consisting of 1-methyl-2-pyrrolidone (NMP), hexamethylphosphorous triamide (HMPT), dimethylsulfoxide (DMSO), and urea derivatives of the following formula: 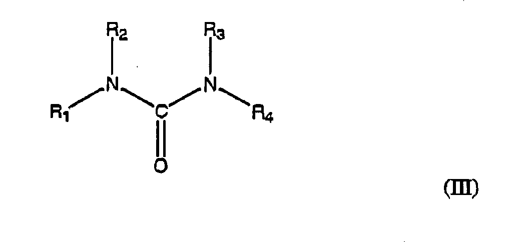 wherein R₁, R₂, R₃ and R₄ may each independently be H or a C₁-C₄ alkyl group and whereby R₂ and R₃ may together form a heterocyclic group.

## Description

The invention relates to a process comprising a condensation step wherein a starting aldehyde or ketone is made to react with an ester of an α-haloacid to form a glycidic ester.

An example of such a process is a process comprising the known Darzens-type condensation reaction, discussed in for example US 2,987,550. In US 2,987,550 the Darzens preparation is disclosed as being a part of the preparation of the so-called 'C₁₄ aldehyde'. In US 2,987,550 and also within the context of the present invention the term 'C₁₄ aldehyde' refers to 2-methyl-4-(2,6,6-trimethyl-1-cyclohexene-1-yl)-2-butenal, an important intermediate in various processes such as the preparation of vitamin A, having the structural formula (I):

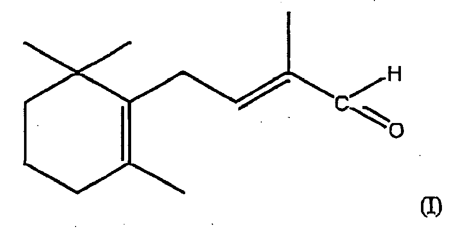

In the known process, the Darzens condensation is carried out by creating a reaction mixture of β-ionone and an excess of a lower alkyl chloroacetate, e.g. methyl or ethyl chloroacetate. As is known, the term β-ionone refers to 4-(2,6,6-trimethyl-1-cyclohexene-1-yl)-3-buten-2-one, a compound having formula (II):

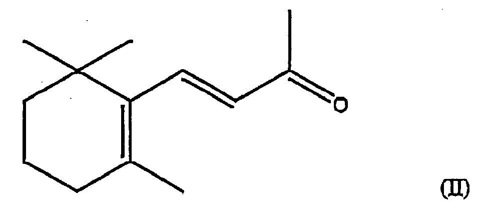

During the mixing of the β-ionone with the alkyl chloroacetate in the known process, an essentially anhydrous diluent is added; the diluent is in the known process chosen from the group consisting of dimethylformamide, pyridine, α-, β-, or γ-picoline, dimethylacetamide and N-erhylacetamide. This group of diluents comprises dipolar aprotic diluents. A dry, alcohol-free alkali metal alkoxide is also added to the reaction mixture. The reaction mixture is subsequently treated with cold methanolic sodium hydroxide at a low temperature, followed by recovery of the C₁₄ aldehyde. Recovery is in the known process typically done by pouring the reaction mixture into water, followed by extraction by an immiscible solvent such as hexane.

The known process has as disadvantage that a high amount of the compound acting as diluent should be used in order to achieve a high yield. As illustration of this, reference is made to examples I and III of US 2,987,550, where a reduction of the molar ratio of the diluent dimethylformamide to β-ionone from about 2.2 in example I to about 1.1 in example III leads to a reduction of yield from 84.8% to 78%. Furthermore, reference is made to Example V. In this example, a yield of 84.6 % was achieved; however, about 5 moles of the diluent pyridine was used per 1.11 mole of β-ionone, i.e. a ratio of about 4.5. Similar excess ratios of diluents are found in the other examples.

It is the objective of the present invention to reduce the disadvantage of the known process.

The said objective is achieved in that the condensation step is carried out in the presence of a dipolar aprotic compound selected from the group consisting of 1-methyl-2-pyrrolidone (NMP), hexamethylfosforic acidtriamid (HMPT), dimethylsulfoxide (DMSO), and urea derivatives of the following formula (III): 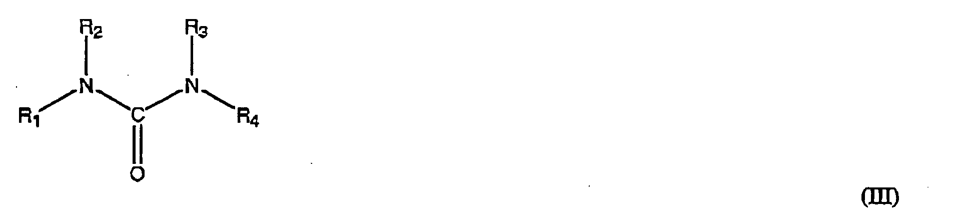 wherein R₁, R₂, R₃ and R₄ may each independently be H or a C₁-C₄ alkyl group and whereby R₂ and R₃ may together form a heterocyclic group.

The process according to the invention has the advantage that very high selectivities and yields can be achieved without having to use high amounts - in relation to the starting aldehyde or ketone - of a further compound such as a compound acting as diluent.

A further advantage of the process according to the invention is that also the molar excess of the ester of α-haloacid - as compared to the starting aldehyde or ketone - can be reduced compared to the known process.

Yet another advantage of the process according to the invention is that the dipolar aprotic compound selected according to the invention can have fewer or even no health- or environmental objections, and fewer objections regarding unpleasant odours, which is important because compounds such as the C₁₄ aldehyde are a.o. used in the preparation of perfumes.

In CN 1348947, a process for the production of C₁₄ aldehyde from β-ionone via a Darzens condensation and using a solvent is disclosed. According to CN 1348947, the solvent should be a compound according to the formula (IV) as given below.

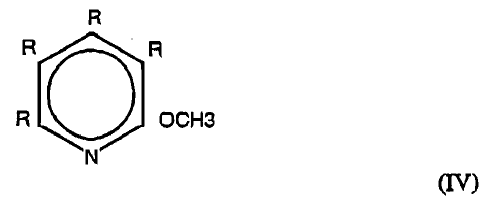

The process according to the invention comprises a condensation step. In this step, a starting aldehyde or a starting ketone are present. Aldhydes and ketones are as such known. In the process according to the invention, a wide variety of aldehydes or ketones may be used; preferably, they are aromatic, An example of a starting ketone is β-ionone, the compound according to formula (II).

In the condensation step, an ester of an α-haloacid is also present. Preferably, the ester of an α-haloacid is methylchloroacetate, ethylchloroacetate, propylchloroacetate or butylchloroacetate; most preferably, the ester of an α-haloacid is methylchloroacetate.

According to the invention, the starting aldehyde or ketone and the ester of an α-haloacid are made to react with each other. This should be done in such a fashion that a glycidic ester is formed. One known method of achieving this is via the known Darzens type of condensation reaction. As is known, in a Darzens condensation reaction the starting aldehyde or ketone is brought together with the ester of an α-haloacid; in order to make these react with each other, an alkali metal alkoxide (preferably dry and alcohol-free) is added; in this fashion, the glycidic ester is formed. As alkali metal alkoxide, sodium methylate is preferred, Generally speaking, the 'alkoxide part' of the alkali metal alkoxide is preferably chosen so that it matches the 'ester part' of the ester of an α-haloacid (e.g., alkali metal *methylate* and the *methyl* ester of an α-haloacid such as chloroacetic acid); this has the advantage that the creation of a mixture of different alcohols is avoided. In a preferred embodiment of the condensation step, β-ionone is the starting ketone, methylchloroacetate is the ester of an α-haloacid and sodium methylate is the alkali metal alkoxide. The glycidic ester thus formed has formula (V):

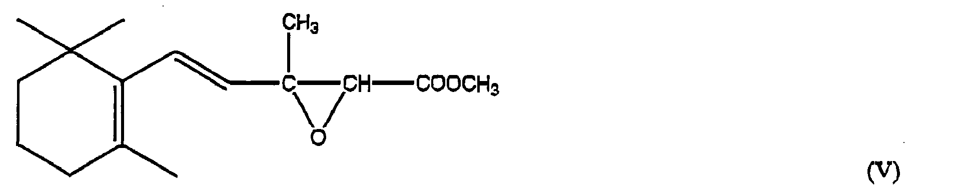

According to the invention, the condensation step is carried out in the presence of a further compound, not being the starting aldehyde or ketone, the ester of an α-haloacid or the alkali metal alkoxide. In contrast to the known process where the said further compound is chosen from the group as detailed in the discussion above on the known process, the further compound is a dipolar aprotic compound or mixture of compounds selected from the group consisting of 1-methyl-2-pyrrolidone (NMP), hexamethylfosforic acidtriamid (HMPT), dimethylsulfoxide (DMSO), and urea derivatives of the following formula: 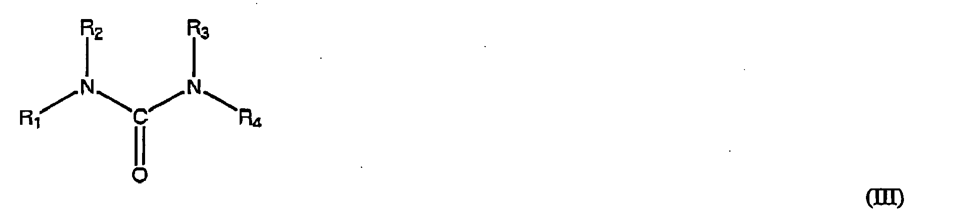 wherein R₁, R₂, R₃ and R₄ may each independently be H or a C₁-C₄ alkyl group and whereby R₂ and R₃ may together form a heterocyclic group. Without committing to scientific theory, it is thought that the further compound according to the invention does not significantly react during the condensation step, but rather may act as diluent and/or as solvent and/or as heat transfer medium and/or in yet another fashion.

As is known, the compound 1-methyl-2-pyrrolidone (NMP) has formula (VI).

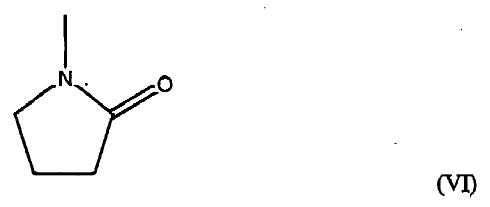

In formula (III), it is preferred that R₁, R₂, R₃ and R₄ are each independently H or a methyl or an ethyl group. In another embodiment, it is preferred that R₁, R₂, R₃ and R₄ are identical. In another class of preferred embodiments of the compound according to formula (III), R₂ and R₃ are such that compound (III) comprises a heterocyclic ring. As is known, the term heterocyclic ring indicates a ring structure wherein the ring-forming atoms are not all carbon. Since R₂ and R₃ will in the case of ring-forming only contribute carbon atoms as ring-forming atoms, this implies that within the context of the present invention the two nitrogen atoms are comprised within the ring structure. If the compound according to formula (III) has a heterocyclic ring, then this ring is preferably a 5- or 6-ring. Preferred examples of compounds according to formula (III) are urea, 1,3-dimethyltetrahydro-2(1H)-pyrimidinone (DMPU) or 1,1,3,3-tetramethylurea (TMU).

The dipolar aprotic compound as selected according to the invention does not need to be in liquid form, in particular not if the starting aldehyde or ketone and/or the ester of an α-haloacid are in liquid form; a liquid form is, nevertheless, preferred. Most preferably, the condensation step is carried out in the presence of NMP.

It is an advantage of the condensation step according to the invention that high yields towards the glycidic ester may be achieved with the utilisation of a relatively limited amount of the further compound such as NMP. It is preferred that the molar ratio in the condensation step between the dipolar aprotic compound - as selected according to the invention - and the starting aldehyde or ketone lies between 0.05 and 2. The said molar ratio is preferably higher than 0.05, so that the desired effect of yield enhancement is clearly noticeable. More preferably, the said ratio is at least 0.08, 0.10,0.12, or 0.15. The said molar ratio is preferably at most 2.00 so as to reap the maximum economical benefit from the condensation step according to the invention. More preferably, the said molar ratio is at most 1.90, 1.80, 1.75, 1.70, or 1.65.

It is an advantage of the condensation step according to the invention that the molar excess of the ester of an α-haloacid - as compared to the starting aldehyde or ketone - can be reduced compared to the known process. This is particularly advantageous since this can be - and preferably is - combined with a low molar ratio between the dipolar aprotic compound as selected according to the invention and the starting aldehyde or ketone, as detailed above. Preferably, the molar ratio between the ester of an α-haloacid and the starting aldehyde or ketone lies between 1.00 and 2.00. More preferably, the said ratio is at least 1.05, 1.10, or 1.15, and at most 1.90, 1.80, or 1.70.

It is a further advantage of the condensation step according to the invention that the molar excess of the alkali metal alkoxide - as compared to the starting aldehyde or ketone - can be reduced compared to the known process. This is particularly advantageous since this can be - and preferably is - combined with a low molar ratio between the dipolar aprotic compound as selected according to the invention and the starting aldehyde or ketone, as detailed above. Even more advantageous is the preferred embodiment of also combining the low amount of alkali metal alkoxide with a low amount of an ester of an α-haloacid, according to the ranges as detailed above, Preferably, the molar ratio between the alkali metal alkoxide and the starting aldehyde or ketone lies between 1.00 and 2.00. More preferably, the said ratio is at least 1.05, 1.10, or 1.15, and at most 1.90, 1.80, or 1.70.

The condensation step according to the invention may typically be carried out at atmospheric pressures; there is usually not a ground to operate at reduced or increased pressures, although this is certainly possible should the characteristics of one of the compounds present require this according to the knowledge of those skilled in the art. It was found furthermore that it is often beneficial to carry out the condensation step according to the invention in a temperature range lying between -20°C and +10°C, as in this range both selectivity and yield are good. Within this range, the selectivity towards glycidic ester formation tends to increase as the temperature decreases. More preferably, the said temperature range goes from -15°C to +5°C.

It is preferred that in the condensation step according to the invention the compounds designated as diluents or solvents in the known processes such as those of US 2,987,550 or CN 1348947 are present in only limited amounts, i.e. less than 50 wt.%, 40, 30, or even less than 10% compared to the amount of the dipolar aprotic compound selected according to the invention. More preferably, the said compounds according to the known processes are essentially absent.

In practice and preferred according to the present invention, the condensation step is followed by a saponification step. In the saponification step, the principle of which is as such known, the glycidic ester as formed in the condensation step is saponified. The said saponification may be achieved in a known fashion, e.g. by bringing the glycidic ester in contact with sodium hydroxide, whereby the said sodium hydroxide is typically anhydrous, and often dissolved in an alcohol such as methanol. According to a preferred embodiment according to the invention, the saponification step is done on the glycidic ester according to formula (V).

In practice and also preferred according to the present invention, the saponification step is followed by a hydrolysis step. In this step, the saponified glycidic ester is brought into contact with an aqueous phase; this leads to decarboxylation of the saponified glycidic ester, whereby an aldehyde or ketone is formed. According to a preferred embodiment according to the invention, the hydrolysis step is done on the glycidic ester according to formula (V) as saponified in the saponification step; the resulting aldehyde is then the C₁₄ aldehyde according to formula (I).

Upon completion of the hydrolysis step, an aqueous system is present comprising water, the aldehyde or ketone as formed, and also the dipolar aprotic compound as selected according to the invention. The aldehyde or ketone as formed may be recovered with methods known to the skilled person. An example of such a method is an extraction step in which the aqueous system is brought in contact with an extracting agent, said extracting agent being limitedly or essentially non-miscible with water, and chosen such that the aldehyde or ketone migrates preferentially into the extracting agent. Examples of extracting agents are alkanes such as hexane.

The invention will be illustrated by means of the following examples, without being limited thereto.

### Example 1

β-ionone (212.7 g, 97% purity, 1.07 moles), methylchloroacetate (146.4 g, 1.35 moles) and NMP (75 g, 0.76 moles) were put into a stirred reactor and cooled to 0°C. Sodium methylate (87.6 g, 1.62 moles) was then dosed, leading to the reaction to the corresponding glycid ester. After the sodium methylate had been added, the reactor contents were brought to 20°C and remained there for 20 minutes. In this Example, the molar ratio of NMP to β-ionone was 0.71; the molar ratio of methlychloroacetate to β-ionone was 1.23; the molar ratio of sodium methylate to β-ionone was 1.51.

Subsequent to the formation of the glycidic ester, saponification was achieved through addition of sodium hydroxide, whereby the temperature rose to 35°C. The reaction mixture was then transferred to a second stirred vessel; in the said second stirred vessel, the hydrolysis reaction to the C₁₄ aldehyde was done by means of addition of water. Then, hexane was added to the reaction mixture - while being stirred. This lead to the extraction of the C₁₄ aldehyde from the aqueous mixture into the hexane phase. The hexane phase was separated from the aqueous phase and acidified through the addition of some aqueous acetic acid. The C₁₄ aldehyde was then isolated through evaporation of the hexane under vacuum followed by distillation. A total of 205.6 g of the C₁₄ aldehyde was recovered, corresponding to a yield of 89.4% (calculated from the raw material β-ionone).

### Examples 2 - 5

The procedure of Example 1 was repeated, the only difference being the amount of NMP that was added. The results are given in the table below.

| Example | Amount of NMP (g) | Molar ratio NMP/β-ionone (-) | Yield of C₁₄ aldehyde (%) |
|---|---|---|---|
| 2 | 15 | 0.14 | 84.4 |
| 3 | 45 | 0.42 | 88.5 |
| 4 | 120 | 1.13 | 88.9 |
| 5 | 165 | 1.56 | 85.5 |

### Examples 6 - 10

The procedure of Example 1 was repeated; however, instead of NMP, DMPU was added in various amounts. The results are given in the table below.

| Example | Amount of DMPU (g) | Molar ratio DNIPU/β-ionone (-) | Yield of C₁₄ aldehyde (%) |
|---|---|---|---|
| 6 | 15 | 0.11 | 86.0 |
| 7 | 45 | 0.33 | 89.0 |
| 8 | 75 | 0.55 | 87.6 |
| 9 | 120 | 0.88 | 86.0 |
| 10 | 165 | 1.20 | 84.4 |

### Examples 11-15

The procedure of Example 1 was repeated; however, instead of NMP, TMU was added in various amounts. The results are given in the table below.

| Example | Amount of TMU (g) | Molar ratio TMU/ β-ionone (-) | Yield of C₁₄ aldehyde (%) |
|---|---|---|---|
| 11 | 15 | 0.13 | 84.7 |
| 12 | 45 | 0.40 | 88.8 |
| 13 | 75 | 0.66 | 90.0 |
| 14 | 120 | 1.06 | 90.5 |
| 15 | 165 | 1.46 | 90.4 |

## Claims

1. Process comprising a condensation step wherein a starting aldehyde or ketone is made to react with an ester of an α-haloacid to form a glycidic ester, whereby the reaction is carried out in the presence of a dipolar aprotic compound selected from the group consisting of 1-methyl-2-pyrrolidone (NMP), hexamethylphosphorous triamide (HMPT), dimethylsulfoxide (DMSO), and urea derivatives of the following formula; 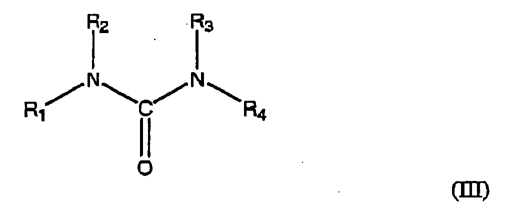 wherein R₁, R₂, R₃ and R₄ may each independently be H or a C₁-C₄ alkyl group and whereby R₂ and R₃ may together form a heterocyclic group.

2. Process according to claim 1, wherein the condensation step is carried out in the presence of an alkali metal oxide.

3. Process according to claim 1 or 2, further comprising a saponification step, wherein the glycidic ester is saponified, and a hydrolysis step, wherein the saponified glycidic ester is decarboxylated to form an aldehyde.

4. Process according to claim 3, wherein the raw materials for the condensation step comprise β-ionone, methylchloroacetate, sodium methylate and 1-methyl-2-pyrroudon (NMP), and whereby in the hydrolysis step the C₁₄ aldehyde 2-methyl-4-(2,6,6-trimethyl-1-cyclohexene-1-yl)-2-butenal is formed.

5. Process according to any one of claims 1-4, wherein the molar ratio in the condensation step between the dipolar aprotic compound and the starting aldehyde or ketone lies between 0.05 and 2.

6. Process according to any one of claims 1-5, wherein the molar ratio in the condensation step between the ester of an α-haloacid and the starting aldehyde or ketone lies between 1.0 and 2.0.

7. Process according to any one of claims 2 - 6, wherein the molar ratio in the condensation step between the alkali metal alkoxide and the starting aldehyde or ketone lies between 1.0 and 2.0.

8. Process according to claim 1, wherein the dipolar aprotic compound is according to formula (III) and is 1,3-dimethyltetrahydro-2(1H)-pyrimidinone (DMPU) or 1,1,3,3-tetramethylurea (TMU).

9. Process for the preparation of vitamin A or an ester of vitamin A, **characterised in that** the process comprises the process according to anyone of claims 1 - 8.
